# EUROPEAN PATENT SPECIFICATION

(11) **EP 1 195 607 B1**
(45) Date of publication and mention of the grant of the patent: **04.05.2011**
(21) Application number: 01124678.2
(22) Date of filing: 09.10.2001
(51) Int. Cl.: G01N 33/68, G01N 33/543, G01N 33/58, G01N 33/558

(54) **Immunological analytical method for the determination of glycosylated protein (AGEs)**
Immunologisch-analytisches Verfahren zur Bestimmung von glykosylierten Proteinen (AGEs)
Méthode d'analyse immunologique pour la détermination des protéines glycosylées (AGEs)

(30) Priority: 09.10.2000 CN 00253607 U
(43) Date of publication of application: 10.04.2002
(60) Divisional application: 06014487.0
(73) Proprietor: Liu, Yung-Hsiang, Taipei, Taiwan (CN); Chan, Wing-yee, Taipei, Taiwan (CN)
(72) Inventor: Liu, Yung-Hsiang, Taipei, Taiwan (CN); Chan, Wing-yee, Taipei, Taiwan (CN)
(74) Representative: Kador & Partner

(56) References cited:
- EP-A- 0 291 194
- EP-A- 0 293 779
- EP-A- 0 315 866
- EP-A- 0 573 972
- EP-A- 0 779 513
- WO-A-97/34147
- FR-A- 2 592 717
- US-A- 5 160 486
- US-A- 5 670 381
- US-A- 5 683 887
- US-A- 6 001 875
- KOPP HP ET AL.: "Evaluation einer neuen Methode zur Bestimmung von glykiertem Hämoglobin mittels monoklonaler Antikörper (DCA 2000)." WIENER KLINISCHE WOCHENZEITSCHRIFT, vol. 108, no. 1, 1996, pages 16-19, XP002200257
- TIRAN A ET AL.: "Photometrische Bestimmung des HbAc1c-Wertes in der Praxis." DEUTSHCE MEDIZINISCHE WOCHENZEITSCHRIFT, vol. 119, no. 23, 1994, pages 833-836, XP002200256
- METUS P ET AL.: "Immunoturbidimetric assay of glycated hemoglobin." JOURNAL OF CLINICAL LABORATORY ANALYSIS, vol. 13, 1999, pages 5-8, XP002200258

## Description

### BACKGROUND OF THE INVENTION

### 1. Field of the invention

The invention relates to an immunological analytical method and the use of reagents for the determination of the glycosylated protein antigen or anti-glycosylated protein antibody, which determine the immunological reaction of the glycosylated protein antigen or anti-glycosylated protein antibody by agglutination phenomenon or accompanied changes of absorbance or color.

### 2. Description of the related art

The pathogenic mechanism of diabetes has been thoroughly understood today. For example, it is know that, under the situation of hyperglycemia, a protein (e.g., albumin) might be glycosylated into glycosylated protein (e.g., Advanced Glycosylated End Products (AGEs)), and this would cause abnormal destruction of cells, particular in a diabetic patient. Further, while the blood sugar level of a diabetic patient can currently be controlled effectively with drugs, the efficient manipulation of complicated condition through earlier prediction is still impossible. Therefore, it has been devoted to develop a specific approach or agent to determine whether the AGE of the diabetes is present or not such that the attending practitioner could prevent the occurrence of the complicated condition in a diabetic patient at the earliest stage, or block the progression of the complicated condition. Among such specific approach or reagent for determining the AGE of the diabetes, the most specific and sensitive one comprises of determining the glycosylated protein antigen or anti-glycosylated protein antibody, such as, for example, the advanced glycosylated end products, by immunological analytic techniques. However, no immunological analytic technique for determining the glycosylated protein antigen or anti-glycosylated protein antibody is available at present.

US-A-5 683 887 (BUCALA RICHARD J, 4 November 1997) describes an ELISA for AGEs In view of the forgoing, the inventor of this application has been studied extensively and finally, developed an immunological analytic technique or devices for determining the AGE antigen or anti-AGE antibody, which essentially comprise of determining the immunological reaction of the glycosylated protein antigen or anti-glycosylated protein antibody by agglutination phenomenon or accompanied changes of absorbance or color, and the invention is thus accomplished.

### SUMMARY OF THE INVENTION

In one aspect, the invention provides an immunological analytic technique for determining the AGE antigen (antibody) with the AGE antibody (antigen), which comprises of determining whether any AGE antigen or antibody is present or not in the sample based on the agglutination phenomenon or accompanied changes of absorbance or color by immunological analytical technique.

In another aspect, the invention provides the use of an immunological analytical reagent for determining the AGE antigen (antibody) with the AGE antibody (antigen), which can determine whether any AGE antigen or antibody is present or not in the sample based on the agglutination phenomenon due to the formation of the immnological complex between, antigen and antibody.

In further another aspect, the invention provides the use of an immunological analytical reagent for determining the AGE antigen (antibody) with the AGE antibody (antigen), which can determine whether any AGE antigen or antibody is present or not in the sample based own AGE antigen or antibody is present or not in the sample based on the change of absorbance due to the formation of the immunological complex between, antibody and antigen (Advanced Glycosylated End Products (AGEs) antigen).

The features, objectives and advantages of the invention will become apparent from perusal of the following description.

### DETAILED DESCRIPTION OF THE REFERRED EMBODIMENT

As described above, the invention relates to the determination of the presence or not of a glycosylated protein, such as the advanced glycosylated end products in a sample to be tested by several immunological analytical techniques. Antibody used in the invention can be those raised immunologically in rabbit or goat with purified antigen (AGE antigen), or those obtained as hybridoma by immunizing mice (e.g., monoclonal antibody).

Immunological analytical techniques are known in the art. For example, Fujikawa H., and Igarashi, H.(Appl. Envir. Microbiol., 54/10, 2345-2348, 1998) disclosed flash emulsion agglutination reagents formed from high density latex granules for detecting enterotoxin A∼E of Staphylococcus. Delanghe, J. R, Chapele, J. P., and Vander schueren, S. C., proposed a colorimetric method for detecting myoglobin (Clin. Chem., 36/9, 1675-1678, 1990). WO 88/08534 (1988) disclosed an immunological analytical device comprising an immunological chromatographic membrane as the medium for determining HCG and LH. US Patent No. 5,236,652 (1993) disclosed an immunological chromatographic technique for determining non-protein antigen.

None of the above-mentioned techniques taught about the immunological analytical technique and device for determining the AGE antigen or AGE antibody disclosed by the invention.

In one aspect, the invention provides the use of an immunological analytic reagent for determining the AGE antigen (antibody) with the AGE antibody (antigen), which comprises of determining whether any glycosylated protein antigen or antibody (Advanced Glycosylated End Products (AGEs) antigen) is present or not in the sample based on an immunological agglutination technique, which comprises:

A displaying carrier emulsion, and the glycosylated protein antigen or anti-glycosylated protein antibody immobilized on the surface of said displaying carrier; which, after contacting said emulsion reagent with a sample to be tested, can determine whether a glycosylated protein antigen or antibody is present or not based on the resultant agglutination phenomenon.

In one embodiment, one or more AGE in antibody is immobilized on said displaying carrier in the emulsion, and a blocking protein is used to fill fully voids in the carrier. When a test sample containing a given amount of glycosylated protein antigen (Advanced Glycosylated End Products (AGEs) antigen) mixes and reacts with a displaying carrier emulsion comprising one or more immobilized antibody on the reacting plate, immunological complexes as "displaying carrier-antibody, or multiple antibodies-AGEs-antibody, or multiple antibodies-displaying carrier....etc.". Such reaction will result into a visible agglutination as a positive response within 3-5 minutes. On the other hand, if no AGE antigen present in the test sample, after mixing with the displaying carrier emulsion comprising one or more immobilized antibody, no agglutination will occur therebetween, and hence is considered as a negative response.

The displaying carrier used in the invention is a fine colored particulate with a particle size of 0.01 - 60 micron. Such particulate may be any of latex micro-particles, dye micro-particles, liposomes, colloidal gold particles, carbon black micro-particles, and the like.

The term "antibody" as used herein refers to one or more of the above-mentioned anti-glycosylated protein antibodies. Immobilization of a glycosylated protein antigen having one structure or a glycosylated protein antigen having various structures can be used to determine whether any antibody of such glycosylated protein antigen is present in the test sample or not; if no agglutination, it is a negative reaction; on the other hand, agglutination means positive reaction.

In another aspect, the invention provides the use of an immunological analytic reagent for determining the AGE antigen (AGE antibody) with the AGE antibody (AGE antigen), which comprises of detecting the glycosylated protein antigen or anti-glycosylated protein antibody (Advanced Glycosylated End Products (AGEs) antigen or antibody thereof) based on an immunological turbidimetric technique, which comprises:

A displaying carrier suspension, immobilized on the surface of said displaying carrier with the above-mentioned glycosylated protein antigen or antibody; and

A device for measuring absorbance;
which, after contacting said reagent with a sample to be tested, can determine whether a glycosylated protein antigen or antibody is present or not based on the resultant change or not of absorbance.

In one embodiment, one or more glycosylated protein antibody is immobilized on said displaying carrier in the emulsion, and a blocking protein is used to fill fully voids in the carrier. A negative serum standard, a weak positive serum standard, and a unknown test sample are prepared, respectively. To three colorimetric tubes A1, A2 and A3 are charged each of 250 micro-liter of displaying carrier emulsion containing immobilized one or more antibodies. Then, adds 20 micro-liter of the negative serum standard into the colorimetric tube A1; adds 20 micro-liter of the weak positive serum standard into the colorimetric tube A2; and adds 20 micro-liter of the unknown sample into the colorimetric tube A3. Immediately after the additions are accomplished, the tubes are subjected to turbidity or absorbance measurement at 340 nm, and recorded the respective optical density (OD) value. At 240 seconds after addition of the sample, the respective OD values are recorded again. The difference by subtracting the first recorded OD values from the second recorded OD values, respectively, is the OD value of the immunological reaction. By comparing three OD differences thus obtained, when the difference of OD value from the unknown sample is greater than that from the weak positive serum standard, it can be considered as a positive response. On the other hand, when the difference of OD value from the unknown sample is less than that from the weak positive serum standard, it can be considered as a negative response. Furthermore, by preparing a series of standard solutions each having a know concentration, such as, for example, 0, 1, 2, 4, 8, and 16 units/ml, the concentration in the test sample can be calculated from the standard curve plotted from data obtained with such standard solutions.

The displaying carrier used herein is a fine particulate. Such particulates may be any of latex micro-particles, liposomes, polyethylene glycol micro-particulates, carbon micro-particles, dye micro-particles, NADH micro-particles, and the like with a particle size of 0.001 - 20 micron and a spectrophotometric range of 260 nm to 840 nm.

The term "antibody" as used herein refers to one or more of the above-mentioned anti-glycosylated protein antibodies. Immobilization of a glycosylated protein antigen having one structure or a glycosylated protein antigen having various structures can be used to determine whether any antibody of such glycosylated protein antigen is present in the test sample or not. Alternatively, a competitive method can used to yield a same experimental result. For example, when conducted in the same manner as described above, additionally, a given amount of glycosylated protein antigen or antibody with known concentration can be used together with each of the test sample, the positive standard solution and the negative standard solution to react respectively the displaying carrier emulsion having the antibody or antigen immobilized thereon, a similar result can be obtained as above.

The invention will be illustrated further more detailed with the following non-limiting examples.

### Example 1

### Raising of the antibody of the glycosylated protein

The antibody of the glycosylated protein is prepared as a polyclonal antibody by immunizing directly a rabbit or a goat with purified antigen, e.g., AGE antigen, or by immunizing mice into hybridoma and further manipulating to yield as a monoclonal antibody.

### Example 2

### The agglutination assay of the glycosylated protein antigen

A polystyrene bead or other colored micro-particles having a particle size of about 0.8 micrometer was used as the displaying carrier micro-particle and was diluted into a concentration of 3%. The AGE antibody obtained in Example 1 above was diluted with a phosphate buffer into a concentration of 2 mg/ml. 10 ml each of the displaying carrier micro-particle suspension and the antibody solution were added into a glass tube and mixed well, which then stood for 8 hours. Thereafter, 1 g of bovine serum albumin (BSA) was added and mixed in the tube, which then stood for 8 hours. After centrifuging at 12000 rpm for 30 minutes, the supernatant was discarded, and repeated this operation once more. 2% BSA solution was added to a total amount of 20 ml. The mixture was sonicated into a homogeneous suspension as the desired displaying carrier suspension.

Three solutions of negative standard serum were prepared as having a AGE antigen content of 0, 1 and 2 units /ml, respectively. A solution of weak positive standard serum was prepared as having a AGE antigen content of 5 units /ml. A solution of strong positive standard serum was prepared as having a AGE antigen content of 16 units /ml. Finally, an unknown test sample was provided.

100 microliter each of the serum solutions prepared above was placed into a test device having the test sample loaded, respectively. 50 microliter of the displaying carrier suspension was added into each test device, and read results after allowing them developing for 3 - 5 minutes.

### Results:

| | The displaying carrier agglutination suspension |
|---|---|
| Negative standard serum 0 unit/ml | - |
| Negative standard serum 1 unit/ml | - |
| Negative standard serum 2.5 unit/ml | - |
| Weak positive standard serum 5 unit/ml | + |
| Strong positive standard serum 16 unit/ml | + |
| Unknown test sample | + |

A agglutination reaction means a positive reaction for the AGE antigen test, while no agglutination reaction means a negative reaction for the AGE antigen test. With the minimum positive reaction limit set at 5 unit/ml, the positive reaction of the unknown test sample indicates the concentration of the AGE antigen in this test sample is ≧ 5 unit/ml.

### Example 3

### Immunological turbidimetric assay of the glycosylated protein

0.2 g of the displaying carrier micro-particles was added into one liter of distilled water to prepare a suspension. The displaying carrier may be a white polystyrene bead having a particle size of about 0.3 micrometer. Alternatively, micro-particles having color at other wavelength can be employed also. To this suspension, 30 mg of the anti-AGE antibody was added and mixed well, and then stood for 18 hours. Next, 4 g of BSA was added and mixed well, and again stood for 18 hours. The mixture was centrifuged at 12000 rpm for 30 minutes and the resulting supernatant was discarded. This process was repeated three times. 2% BSA solution was added to a total amount of one liter. The mixture was sonicated into a homogeneous suspension as the desired displaying carrier suspension.

Six standard serum solutions were prepared as having a AGE antigen content of 0, 1, 2, 4, 8 and 16 units /ml, respectively. An unknown test sample was provided.

250 microliter of the displaying carrier suspension was added into a respective colorimetric tube. 20 micrometer each of the standard serum solution prepared above was added into each colorimetric tube, respectively. 20 microliter of the unknown test sample solution was added into another colorimetric tube.

The colorimeter was set to null with air at a wavelength of 340 nm. When 20 microliter each of the standard serum solution and unknown test sample were added, the OD value of individual colorimetric tube should read immediately and read again at 240 second thereafter. A OD difference of the reaction was calculated by subtracting the first OD value from the second OD value.

With the positive reaction limit set at ≧ 5 unit/ml, each reaction OD value obtained from each standard serum solution was plotted as a standard curve. The concentration of the AGE antigen in the test sample can then be calculated from this standard curve and the resulting value is 9 unit/ml, a positive reaction.

### Results:

| | OD value at 0 sec | OD value at 240 sec | Redaction OD value | Units |
|---|---|---|---|---|
| Standard serum A | 0.86 | 0.85 | 0.01 | 0 |
| Standard serum B | 0.92 | 0.87 | 0.05 | 1 |
| Standard serum C | 0.98 | 0.82 | 0.16 | 2 |
| Standard serum D | 1.02 | 0.83 | 0.19 | 4 |
| Standard serum E | 0.94 | 0.69 | 0.25 | 8 |
| Standard serum F | 0.99 | 0.69 | 0.3 | 16 |
| Unknown test sample G | 1.07 | 0.81 | 0.26 | 9 |

According to this example, negative serum solution having a concentration of 0 unit/ml and a positive serum solution having a concentration of 5 units/ml can be used to conduct a qualitative determination. If the reaction OD value of the unknown test sample is higher than the reaction OD value of the positive standard serum of 5 units/ml, a positive reaction is indicated. Otherwise, it is a negative reaction.

In conclusion, by testing with the specific reagent and the method according to the invention, the presence or not of the AGE in the diabetes can be readily known so that the practitioner can, at the earliest stage, prevent the progression of the complicated conditions in the diabetic patient or blocking the further progression of the complicated conditions.

## Claims

1. Use of a reagent for determining a glycosylated protein antigen or anti-glycosylated protein antibody, wherein the glycosylated protein is an advanced glycosylated end product, comprising:
a displaying carrier suspension, and
an affinity substance immobilized on said displaying carrier;
wherein, after contacting a test sample with said reagent, the presence or not of the glycosylated protein antigen or anti-glycosylated protein antibody in said test sample can be determined based on the occurrence or not of an agglutination phenomenon.

2. Use of a reagent as in claim 1, wherein said displaying carrier is a colored micro-particle.

3. Use of a reagent in claim 1, wherein said displaying carrier has a particle size in the range of 0.01-60 micrometer.

4. Use of a reagent as in claim 1, wherein said affinity substance is a glycosylated protein antigen.

5. Use of a reagent as in claim 1, wherein said affinity substance is a glycosylated protein antigen having various structures.

6. Use of a reagent as in claim 1, where said affinity substance is an anti-glycosylated protein antibody.

7. Use of a reagent as in claim 1, wherein said affinity substance is a multiple anti-glycosylated protein antibody.

8. Use of a reagent for determining a glycosylated protein antigen or anti-glycosylated protein antibody, wherein the glycosylated protein is an advanced glycosylated end product,
comprising:
a displaying carrier suspension, and
an affinity substance immobilized on said displaying carrier;
wherein, after contacting a test sample with said reagent and measuring the absorbance, the presence or not of the glycosylated protein or anti-glycosylated protein antibody in said test sample can be determined based on the change or not of the absorbance, wherein the change of absorbance accompanies agglutination.

9. Use of a reagent as in claim 8, wherein said micro-particle is a colored micro-particle.

10. Use of a reagent as in claim 8, wherein said micro-particle is a micro-particle selected from the group consisting of liposome, dye micro-particle, polyethylene glycol micro-particle, NADH micro-particle, polymeric micro-particle or carbon micro-particle.

11. Use of a reagent as in claim 8, wherein said absorbance of said displaying carrier is measured at a wavelength in the range of 260 nm to 840 nm.

12. Use of a reagent as in claim 8, wherein said affinity carrier has a particle size in the range of about 0.001-20 micrometer.

13. Use of a reagent as in claim 8, wherein said affinity substance is a glycosylated protein antigen.

14. Use of a reagent as in claim 8, wherein said affinity substance is a glycoyslated protein antigen having various structures.

15. Use of a reagent as in claim 8, wherein said affinity substance is an anti-glycosylated protein antibody.

16. Use of a reagent as in claim 8, wherein said affinity substance is a multiple anti-glycosylated protein antibody.

17. Use of a reagent as in claim 8, useful for determining a glycosylated protein antigen or an anti-glycosylated protein antibody.

18. A method for determining glycosylated protein antigen or anti-glycosylated protein antibody, wherein the glycosylated protein is an advanced glycosylated end product, comprising essentially of using a suspension containing a displaying carrier which has an affinity substance immobilized thereon; mixing a test sample with said suspension, and determining whether a glycosylated protein or anti-glycosylated protein antibody is present or not based on the occurrence of an agglutination phenomenon in said suspension.

19. A method as in claim 18, wherein said affinity substance is an anti-glycosylated protein antibody.

20. A method as in claim 18, wherein said affinity substance is a glycosylated protein antigen.

21. A method for determining glycosylated protein antigen or anti-glycosylated protein antibody, wherein the glycosylated protein is an advanced glycosylated end product, comprising essentially of using a suspension containing a displaying carrier which has an affinity substance immobilized thereon, mixing an appropriate amount of said displaying carrier suspension with a test sample, a positive standard solution and a negative standard solution, respectively, in difference colorimetric tubes, measuring on a proper absorbance measuring device the respective change of absorbance and determining the results through turbidimetric method, wherein the change of absorbance accompanies agglutination.

22. A method as in claim 21, wherein said affinity substance is an anti-glycosylated protein antibody.

23. A method as in claim 21, wherein said affinity substance is an anti-glycosylated protein antigen.

## Patentansprüche

1. Verwendung eines Reagenz zum Bestimmen eines Antigens für glycosyliertes Protein oder eines Antikörpers für glycosyliertes Protein, wobei das glycosylierte Protein ein hochentwickeltes glycosyliertes Endprodukt ist, umfassend:
eine Suspension eines anzeigenden Trägers und
eine auf dem anzeigenden Träger immobilisierte Affinitätssubstanz;
wobei nach dem Inkontaktbringen einer Testprobe mit dem Reagenz das Vorhandensein oder Nichtvorhandensein des Antigens für glycosyliertes Protein oder des Antikörpers für glycosyliertes Protein in der Testprobe auf der Basis dessen bestimmt werden kann, ob das Agglutinationsphänomen auftritt oder nicht.

2. Verwendung eines Reagenz nach Anspruch 1, wobei der anzeigende Träger ein gefärbtes Mikropartikel ist.

3. Verwendung eines Reagenz nach Anspruch 1, wobei der anzeigende Träger eine Partikelgröße im Bereich von 0,01 bis 60 µm hat.

4. Verwendung eines Reagenz nach Anspruch 1, wobei die Affinitätssubstanz ein Antigen für glycosyliertes Protein ist.

5. Verwendung eines Reagenz nach Anspruch 1, wobei die Affinitätssubstanz ein Antigen für glycosyliertes Protein mit unterschiedlichen Strukturen ist.

6. Verwendung eines Reagenz nach Anspruch 1, wobei die Affinitätssubstanz ein Antikörper für glycosyliertes Protein ist.

7. Verwendung eines Reagenz nach Anspruch 1, wobei die Affinitätssubstanz ein multipler Antikörper für glycosyliertes Protein ist.

8. Verwendung eines Reagenz zum Bestimmen eines Antigens für glycosyliertes Protein oder eines Antikörpers für glycosyliertes Protein, wobei das glycosylierte Protein ein hochentwickeltes glycosyliertes Endprodukt ist, umfassend:
eine Suspension eines anzeigenden Trägers und
eine auf dem anzeigenden Träger immobilisierte Affinitätssubstanz;
wobei nach dem Inkontaktbringen einer Testprobe mit dem Reagenz das Vorhandensein oder Nichtvorhandensein des Antigens für glycosyliertes Protein oder des Antikörpers für glycosyliertes Protein in der Testprobe auf der Basis einer Veränderung oder keiner Veränderung der Absorption bestimmt werden kann, wobei die Änderung der Absorption die Agglutination begleitet.

9. Verwendung eines Reagenz nach Anspruch 8, wobei das Mikropartikel ein gefärbtes Mikropartikel ist.

10. Verwendung eines Reagenz nach Anspruch 8, wobei das Mikropartikel ein Mikropartikel ist, das aus der Gruppe von Liposom, Farbstoffmikropartikel, Polyethylenglycol-Mikropartikel, NADH-Mikropartikel, polymerem Mikropartikel oder Kohlenstoffmikropartikel ausgewählt ist.

11. Verwendung eines Reagenz nach Anspruch 8, wobei die Absorption des anzeigenden Trägers bei einer Wellenlänge im Bereich von 260 bis 840 nm gemessen wird.

12. Verwendung eines Reagenz nach Anspruch 8, wobei der Affinitätsträger eine Partikelgröße im Bereich von etwa 0,001 bis 20 µm aufweist.

13. Verwendung eines Reagenz nach Anspruch 8, wobei die Affinitätssubstanz ein Antigen für glycosyliertes Protein ist.

14. Verwendung eines Reagenz nach Anspruch 8, wobei die Affinitätssubstanz ein Antigen für glycosyliertes Protein mit unterschiedlichen Strukturen ist.

15. Verwendung eines Reagenz nach Anspruch 8, wobei die Affinitätssubstanz ein Antikörper für glycosyliertes Protein ist.

16. Verwendung eines Reagenz nach Anspruch 8, wobei die Affinitätssubstanz ein multipler Antikörper für glycosyliertes Protein ist.

17. Verwendung eines Reagenz nach Anspruch 8, das bei der Bestimmung eines Antigens für glycosyliertes Protein oder eines Antikörpers für glycosyliertes Protein nützlich ist.

18. Verfahrenzum Bestimmen eines Antigens für glycosyliertes Protein oder eines Antikörpers für glycosyliertes Protein, wobei das glycosylierte Protein ein hochentwickeltes glycosyliertes Endprodukt ist, umfassend im wesentlichen die Verwendung einer Suspension, die einen anzeigenden Träger enthält, der eine darauf immobilisierte Affinitätssubstanz aufweist, das Mischen einer Testprobe mit der Suspension und das Bestimmen, ob ein glycosyliertes Protein oder ein Antikörper für glycosyliertes Protein vorhanden ist oder nicht, und zwar auf der Basis des Auftretens eines Agglutinationsphänomens in der Suspension.

19. Verfahren nach Anspruch 18, wobei die Affinitätssubstanz ein Antikörper für glycosyliertes Protein ist.

20. Verfahren nach Anspruch 18, wobei die Affinitätssubstanz ein Antigen für glycosyliertes Protein ist.

21. Verfahrenzum Bestimmen eines Antigens für glycosyliertes Protein oder eines Antikörpers für glycosyliertes Protein, wobei das glycosylierte Protein ein hochentwickeltes glycosyliertes Endprodukt ist, umfassend im wesentlichen die Verwendung einer Suspension, die einen anzeigenden Träger enthält, der eine darauf immobilisierte Affinitätssubstanz aufweist, das Mischen einer geeigneten Menge der Suspension des anzeigenden Trägers mit einer Testprobe, einer positiven Standardlösung bzw. einer negativen Standardlösung in unterschiedlichen Kolorimetriröhrchen, das Messen der entsprechenden Änderung der Absorption mit einer geeigneten Absorptionsmeßvorrichtung und Bestimmen der Ergebnisse durch ein Trübungsmeßverfahren, wobei die Änderung der Absorption die Agglutination begleitet.

22. Verfahren nach Anspruch 21, wobei die Affinitätssubstanz ein Antikörper für glycosyliertes Protein ist.

23. Verfahren nach Anspruch 21, wobei die Affinitätssubstanz ein Antigen für glycosyliertes Protein ist.

## Revendications

1. Utilisation d'un réactif pour déterminer un antigène de protéine glycosylée ou un anticorps anti-protéine glycosylée, dans laquelle la protéine glycosylée est un produit final glycosylé avancé, comprenant :
une suspension de support de présentation, et
une substance d'affinité immobilisée sur ledit support de présentation ;
dans laquelle, après mise en contact d'un échantillon de test avec ledit réactif, la présence ou l'absence de l'antigène de protéine glycosylée ou de l'anticorps anti-protéine glycosylée dans ledit échantillon de test peut être déterminée sur la base de l'apparition ou de la non-apparition d'un phénomène d'agglutination.

2. Utilisation d'un réactif selon la revendication 1, dans laquelle ledit support de présentation est une microparticule colorée.

3. Utilisation d'un réactif selon la revendication 1, dans laquelle ledit support de présentation a une granulométrie située dans la plage allant de 0,01 à 60 micromètre.

4. Utilisation d'un réactif selon la revendication 1, dans laquelle ladite substance d'affinité est un antigène de protéine glycosylée.

5. Utilisation d'un réactif selon la revendication 1, dans laquelle ladite substance d'affinité est un antigène de protéine glycosylée ayant diverses structures.

6. Utilisation d'un réactif selon la revendication 1, dans laquelle ladite substance d'affinité est un anticorps anti-protéine glycosylée.

7. Utilisation d'un réactif selon la revendication 1, dans laquelle ladite substance d'affinité est un anticorps anti-protéine glycosylée multiple.

8. Utilisation d'un réactif pour déterminer un antigène de protéine glycosylée ou un anticorps anti-protéine glycosylée, dans laquelle la protéine glycosylée est un produit final glycosylé avancé, comprenant :
une suspension de support de présentation, et
une substance d'affinité immobilisée sur ledit support de présentation ;
dans laquelle, après mise en contact d'un échantillon de test avec ledit réactif et mesure de l'absorbance, la présence ou l'absence de l'antigène de protéine glycosylée ou de l'anticorps anti-protéine glycosylée dans ledit échantillon de test peut être déterminée sur la base du changement ou du non-changement de l'absorbance, et dans laquelle le changement d'absorbance accompagne une agglutination.

9. Utilisation d'un réactif selon la revendication 8, dans laquelle ladite microparticule est une microparticule colorée.

10. Utilisation d'un réactif selon la revendication 8, dans laquelle ladite microparticule est une microparticule choisie dans le groupe constitué par un liposome, une microparticule de colorant, une microparticule de polyéthylèneglycol, une microparticule de NADH, une microparticule polymère ou une microparticule de carbone.

11. Utilisation d'un réactif selon la revendication 8, dans laquelle ladite absorbance dudit support de présentation est mesurée à une longueur d'onde située dans la plage allant de 260 nm à 840 nm.

12. Utilisation d'un réactif selon la revendication 8, dans laquelle ladite substance d'affinité a une granulométrie située dans la plage allant d'environ 0,001 à 20 micromètres.

13. Utilisation d'un réactif selon la revendication 8, dans laquelle ladite substance d'affinité est un antigène de protéine glycosylée.

14. Utilisation d'un réactif selon la revendication 8, dans laquelle ladite substance d'affinité est un antigène de protéine glycosylée ayant diverses structures.

15. Utilisation d'un réactif selon la revendication 8, dans laquelle ladite substance d'affinité est un anticorps anti-protéine glycosylée.

16. Utilisation d'un réactif selon la revendication 8, dans laquelle ladite substance d'affinité est un anticorps anti-protéine glycosylée multiple.

17. Utilisation d'un réactif selon la revendication 8, utile pour déterminer un antigène de protéine glycosylée ou un anticorps anti-protéine glycosylée.

18. Procédé pour déterminer un antigène de protéine glycosylée ou un anticorps anti-protéine glycosylée, dans lequel la protéine glycosylée est un produit final glycosylé avancé, comprenant essentiellement l'utilisation d'une suspension contenant un support de présentation sur lequel est immobilisée une substance d'affinité ; le mélange d'un échantillon de test avec ladite suspension, et la détermination de la présence ou de l'absence d'une protéine glycosylée ou d'un anticorps anti-protéine glycosylée sur la base de l'apparition ou de la non-apparition d'un phénomène d'agglutination dans ladite suspension.

19. Procédé selon la revendication 18, dans lequel ladite substance d'affinité est un anticorps anti-protéine glycosylée.

20. Procédé selon la revendication 18, dans lequel ladite substance d'affinité est un antigène de protéine glycosylée.

21. Procédé pour déterminer un antigène de protéine glycosylée ou un anticorps anti-protéine glycosylée, dans lequel la protéine glycosylée est un produit final glycosylé avancé, comprenant essentiellement l'utilisation d'une suspension contenant un support de présentation sur lequel est immobilisée une substance d'affinité, le mélange d'une quantité appropriée de ladite suspension de support de présentation avec un échantillon de test, une solution étalon positive et une solution étalon négative, respectivement, dans différents tubes colorimétriques, la mesure, sur un dispositif approprié de mesure d'absorbance, du changement respectif d'absorbance, et la détermination des résultats par turbidimétrie, dans lequel le changement d'absorbance accompagne une agglutination.

22. Procédé selon la revendication 21, dans lequel ladite substance d'affinité est un anticorps anti-protéine glycosylée.

23. Procédé selon la revendication 21, dans lequel ladite substance d'affinité est un antigène anti-protéine glycosylée.
